Europäisches Patentamt

European Patent Office

Office européen des brevets

(11) Publication number: **0 272 149 B1**

## EUROPEAN PATENT SPECIFICATION

(45) Date of publication of patent specification: **11.03.92**

(51) Int. Cl.5: **A61L 25/00**

(21) Application number: **87311209.8**

(22) Date of filing: **18.12.87**

The file contains technical information submitted after the application was filed and not included in this specification

(54) **Medical dresssings.**

(30) Priority: **19.12.86 DK 6169/86**

(43) Date of publication of application:
**22.06.88 Bulletin  88/25**

(45) Publication of the grant of the patent:
**11.03.92 Bulletin  92/11**

(84) Designated Contracting States:
**DE ES FR GB SE**

(56) References cited:
**EP-A- 0 055 023**
**EP-A- 0 072 251**
**FR-A- 2 392 076**
**GB-A- 2 089 351**
**US-A- 3 339 546**

(73) Proprietor: **COLOPLAST A/S
4, Bronzevej
DK-3060 Espergaerde(DK)**

(72) Inventor: **Samuelsen, Peter
18, Bukkeballevej
DK-2960 Rungsted Kyst(DK)**

(74) Representative: **Watkins, Arnold Jack et al
European Patent Attorney Frank B. Dehn &
Co. Imperial House 15-19 Kingsway
London WC2B 6UZ(GB)**

Rank Xerox (UK) Business Services

# Description

The present invention relates to a medical dressing comprising a physiologically active substance, optionally in a form so as to assist at attaching incontinence or ostomy equipment, drains and/or means for collecting wound secretions to the skin and sealing around the equipment, the dressing being of the kind mainly consisting of (a) a sealing material comprising (i) a continuous phase comprising a tackifier and, e.g., made from an elastomer, a plasticizer for elastomers, a tackifying resin and optionally an oil-based extender and an antioxidant, and (ii) a discontinuous phase dispersed therein and consisting of one or more water-soluble or water-swellable hydrocolloids, starch or cellulose derivatives or hydrophilic polymers, and (b) a non-adhesive water-tight film or other water-tight material placed on that surface of the dressing which is not intended to face the skin of the patient.

Various dressings or skin barriers of this kind are known. One kind is known from DK Patent Specification No. 147,035 corresponding to US Patent Specification No. 4,367,732. According to this known technique the actual tackifier material is a polymer or copolymer of cyclopentadiene, dicyclopentadiene, $\alpha$-pinene and/or $\beta$-pinene and the elastomer is a styrene-olefin-styrene block copolymer. A plasticizer for the styrene blocks thereof is present which lowers their glass transition temperature, and the water-tight film is elastic. In the present context the film does not need to be elastic, and the plasticizer does not need to be present. In connection with the invention also other elastomers may be used, e.g. ethylene-propylene block copolymers (DK Patent Specification No. 147,034 corresponding to US Patent Specification No. 4,231,369), natural rubber and synthetic resins of a similar nature as natural rubber, and silicone rubbers. Polyisobutylene is often used as tackifier resin in such dressings, for instance in US Patent Specification No. 3,339,546 according to which the bandage on one surface is provided with a water-insoluble viscous, gum-like elastic binder, typically polyisobutylene, admixed with a water-soluble or water-swellable hydrocolloid. Here, however, it is the question of a simple blend of the two components, not a dispersion of the latter in the former.

In DK 147,035/US 4,367,732 it is stated that additives having various skin-friendly and/or therapeutic purposes may be incorporated, e.g. pH controlling systems, bactericides, fungicides, or proteins such as collagen, which contribute to a rapid healing, into the continuous phase or as a further discontinuous phase.

From DK Patent Specification No. 109,225 a dressing is known which consists of a water-insoluble film having attached to one surface thereof a blend of a water-soluble hydrophilic colloid and a viscous binder. A medicament may be incorporated into the mixture or applied onto its surface, for instance an antibiotic, an anaesthetic or an anti-inflammatory agent. It is thus the question of a mere blend of the components in which the medicament, if any, is incorporated into the adhesive matrix together with the hydrocolloid and without having any special relationship thereto.

From US Patent Specification No. 4,307,717 a bandage is known which includes a backing element of for instance a plastics material, and attached thereto a matrix having adhesive properties and comprising a solid phase of, e.g., polyacrylamide or polyacrylate and a liquid phase consisting of a solution or emulsion of carbohydrates and/or proteins; the matrix contains a medicament which according to the specification is molecularly dispersed therein. It also appears that the medicament has been dispersed into the solid part of the matrix and that the purpose of using a hydrophilic matrix is to permit it to be sterilized.

From EP Patent Publication No. 55,023 A3 a tackifier mixture is known which comprises an antiseptic selected amongst iodofores, phenolic compounds, mercury-containing compounds, nitrofurazone and chlorohexidine. The tackifier mixture contains 30-60% b.w. of a natural or synthetic viscous rubbery substance and 30-65% b.w. of water-soluble or water-swellable hydrocolloids. The publication states that the composition is prepared by mixing the hydrocolloid and the antiseptic materials and then adding the viscous substance and continuing the mixing until a homogenous, dough-like mass is formed. This must mean that the materials are kneaded until there is obtained an even dispersion of the hydrocolloid and an even dispersion or solution of the antiseptic in the viscous material, typically polyisobutylene, since the hydrocolloid is incompatible with polyisobutylene. It is further stated that the invention described arises from the discovery that by incorporating an antiseptic or germicide one reduces the potential for bacterial growth without interfering in the healing qualities of the dressing. Thus, the purpose seems mainly to be to protect the tackifier composition itself, and this purpose is supported by the described method of preparing it, as the antiseptic will be released very slowly to the skin or wound and presumably at an even rate.

It should finally be mentioned that an occlusive dressing and granules for the treatment of wounds are known from DK patent application No. 1757/83. The dressing comprises a first adhesive layer adapted to cooperate with the liquid flowing from the wound and forming a liquid-tight adhesion to the healthy skin around the wound; a second adhesive layer which forms an aggressive bond by being compressed with the first one; a layer of foam material having semi-open cells, cast on the second adhesive layer, and a cover layer of for instance a polymeric film. The first adhesive layer is composed of pressure-sensitive adhesives, e.g. of the same kinds as mentioned above (polyisobutylene, natural rubber, silicone rubber, acrylonitrile rubber are mentioned), optionally one or more thermoplastic elastomers (butyl rubber, polyisobutylenes of medium molecular weight, copolymers thereof with isoprene and styrene-olefine-styrene copolymers are mentioned) and 10-65% b.w. of water-dispersible hydrocolloids. The first adhesive layer is produced by preparing a homogenous dispersion of the pressure-sensitive adhesive and the thermoplastic elastomer(s) and then admixing the hydrocolloids until a homogenous paste is formed. The latter may further contain small amounts of antioxidant, deodorant, perfumes and pharmacologically active components, e.g. antibiotics, antiseptics and antiinflammatory agents. From the method described in the application it appears that the fine structure of the first adhesive layer must be of the same nature as mentioned hereinabove in connection with EP 55023, and that pharmaceutics or antiseptics possibly present must be released slowly as they are not dissolved or dispersed directly in a hydrocolloid.

This known dressing may according to the specification be combined with a separate granulate the granules of which are placed directly on the wound (especially if it is strongly suppurating) and are covered by and maintained in place by the occlusive dressing. These granules have a size of 0.4-2 mm and consist of one or more water-dispersible hydrocolloids and optionally up to 50% b.w. of water-swellable cohesion-increasing agents and/or hydratable polymers. The granulate may also contain active substances such as antioxidants, deodorants, perfumes or pharmacologically active substances such as antiseptics, antibiotics or antiinflammatory agents.

It thus appears that possible active substances in dressings according to the known technique are present in a solid matrix and are released therefrom directly to the skin and the wound covered by the dressing. This release is supposed to take place at a largely constant rate or a gradually declining rate due to the fact that the parts of the matrix adjacent the skin and the would will eventually be emptied of active substance.

Only in the case of using the granulate described in DK Patent Application No. 1757/83 will there be a direct association between the hydrocolloid particles and the active, especially pharmacologically active substance, and the granulate is intended for use in the treatment of ulcera from which large amounts of exudate are flowing, the granulate being applied to the wound before the occlusive dressing is applied. The granulate is stated to interact with the wound exudate to form a gel-like mass which will prevent any flow through the dressing. The granulate may be removed by rinsing with saline and gradually, as the healing of the wound progresses, the dressing is used without granulate.

The use of such separate granulate is troublesome and time-consuming for the nursing staff; and if the dressing has first been applied without granulate and the wound then starts to suppurate, the dressing must be removed, the granulate applied and a new dressing mounted. If the active substance is not contained in the granules but is dusted onto them, it will be easily separated during storage.

There are many cases in which the continuous, possibly somewhat declining release of active substance from the matrix of a dressing to a wound is appropriate and in such cases the known technique discussed above as a whole can be considered satisfactory.

In other cases, however, it is desirable that the release of the active substance to the wound be dependent on the degree of suppuration of the wound, i.e. the amount of moisture the wound liberates per unit of time. This is especially the case when the active substance is an antiseptic as antiseptics impede the healing of wounds, and it is therefore important that they are released only when they are needed, their importance being greatest under moist conditions as a moist environment will provide the best conditions for bacterial growth.

According to one aspect of the present invention there is provided a dressing which has a physiologically active substance incorporated therein and which comprises:

(a) a sealing material comprising

(i) a continuous phase comprising a tackifier, and

(ii) a discontinuous phase dispersed in the continuous phase and comprising one or more water-souble or water-swellable hydrocolloids, starches or cellulose derivatives or hydrophilic polymers, and

(b) a non-adhesive water-tight film or other water-tight cover material on the surface of the dressing which in use is to be remote from the skin of the patient to which the dressing is applied, the physiologically active substance being incorporated in the discontinuous phase.

The active substances have a low rate of diffusion in the hydrocolloid or other hydrophilic substances applied, and in consequence of this they are not released to the wound or are only released thereto insignificantly when the wound is dry. If, however, the wound starts to suppurate, the discontinuous phase of the hydrocolloid or other hydrophilic material will swell or even be dissolved and the active substance hence is released to the wound where it will exert its normal function. Accordingly, there is caused a kind of automatic control of the release of the active substance exactly dependent on the condition calling for the release.

The most common situation will be that the wound is suppurating most in the beginning and will suppurate less and less as the healing progresses. In this case the effect of the active substance in the disperse phase will set in immediately, i.e. at the most some few minutes after the dressing has been applied, and the time-consuming process of firstly placing a separate granulate on the wound is avoided. At the same time the absorption of water in the hydrocolloid facilitates the removal of the dressing when a rather large amount of water has been absorbed, notably its removal from sore wound areas or mucous membranes without appreciable pain to the patient, after which a new dressing of the same kind can be applied.

From the above it will be understood that the principle of the dressing according to the invention is particularly important when the active substance is an antiseptic as antiseptics may impede the healing of wounds. An important embodiment of the dressing according to the invention is therefore characterized in that an antiseptic is incorporated as an active substance in the discontinuous phase of the dressing.

As examples of suitable antiseptics may be mentioned povidone-iodine (a complex of iodine and a 1-ethenyl-2-pyrrolidinone homopolymer), chlorohexidine, sulfadiazine silver and chloramine. Antibiotics such as neomycine, amphotericine or the tetracyclines may also be used.

Moreover, the principle of the dressing is particularly important in case of inflammatory conditions where it is intended to apply an antiinflammatory agent to the wound. Inflammation of wounds normally causes a considerable liberation of liquid and an antiinflammatory agent present in the disperse hydrophilic phase of the dressing will therefore be rapidly released to the wound and counteract the inflammation. In a further valuable embodiment therefore an antiinflammatory agent may be incorporated into the disperse phase of the dressing as an active substance. As examples of suitable antiinflammatory agents may be mentioned the anti-inflammatory steroids such as hydrocortisone and triamcinolone acetonide. Of course both an antiseptic and an antiinflammatory agent may be present.

One or more other active substances may be present in the disperse phase together with or instead of an antiseptic and/or antibiotic. Examples of such substances are anaesthetics or local anaesthetics such as benzocaine, lidocaine and bupivacaine; various enzymes, notably proteolytic enzymes, collagenases and lysozyme; biologically active proteins such as fibronectins ($\alpha_2$-glucoproteins, $\alpha_2$-opsonins), epidermal growth factor (EGF; often named EGF-urogastron because of the considerable structural similarity to urogastrone) and blood cell growth factor; certain prostaglandins; and simpler nutrients such as vitamins, amino acids and trace metals.

As adhesive material in the continuous phase (i) of the dressing it may contain one or more of the adhesive and sometimes also elastomeric materials known for such purposes and mentioned hereinbefore, such as polyisobutylene, natural rubber, synthetic rubbers, silicone rubbers and gel-like hydrocarbon tackifier resins such as polymers or copolymers of cyclopentadiene, dicyclopentadiene, $\alpha$-pinene and/or $\beta$-pinene.

Other materials known for the purpose may be present in the continuous phase (matrix), notably physically cross-linked copolymers such as styrene-isoprene-styrene block copolymers or ethylene-propylene block copolymers; or random copolymers (not block copolymers) of such or similar monomers.

Examples of useful hydrocolloids for the disperse phase are carboxymethyl starches and carboxymethyl celluloses (which are normally imployed as metal salts and especially sodium salts thereof such as Na-CMC), polyvinyl alcohol, gelatin, pectin and natural rubbers such as guar gum, gum arabic, locust bean gum and karaya as well as high molecular weight polyethylene glycol ("Carbowax") and carboxy polymethylene. As a hydrophilic material in the disperse phase may also be used hydrophilic polymers such as polyacrylates and polyacrylamides.

The layer of sealing material (a), i.e. including the disperse phase, will typically have a thickness of the order of 0.1-3 mm but may be thicker when used for highly suppurating wounds and thinner towards the healing of the wound.

The particle size of the hydrophilic material (ii) may for instance be 25-150 $\mu$m.

The continuous phase (i) of the adhesive material may comprise for instance 40-90% b.w. of the total adhesive layer (a), preferably 40-60% b.w. thereof, whereas the discontinuous phase (ii) suitably comprises 30-60% b.w. of the adhesive layer (a). In case of use for highly suppurating wounds the amount of disperse phase may be increased.

The water-tight film may be of any kind of watertight and waterproof plastics sheets normally used for such purposes and need not possess special properties; it may be elastic or in-elastic. Typically, the film will have a thickness of 10-50 µm. The film may be substituted by a foam material.

The dressing may be in the form of sheets or strips which are cut to fit the intended purpose. It may also be in the form of ready-cut pieces (which like plasters may be available in different sizes), and they may be of any desired shape, for instance circular, elliptic or rectangular. They may also be annular for use in connection with for instance ostomy or drainage equipment. When the dressing is in the form of intact or annular pieces having a definite size, the layer of sealing material (a) may be bevelled as described in European Patent Application No. 87.309180.5.

On the side of the dressing opposite the water-tight film (b) a protective cover will normally be applied by the manufacturer.

The dressing may be in the form of a laminate in which only the part adjacent the wound or skin has the structure described or in which only that part contains the active substance.

The invention also relates to a method of producing a dressing as hereinbefore described, which comprises dissolving or dispersing a physiologically active substance evenly in a solution, preferably aqueous, of the substance or substances forming the remainder of the discontinuous phase of the sealing material, subsequently drying the solution (for instance by spray-drying) and, if necessary, comminuting the dried material into a desired particle size and sieving it to a desired particle size distribution, then kneading or otherwise incorporating the dry particles, (for instance by forceful gas pressure) into a molten or heat-softened mass of the components forming the continuous phase of the sealing material, and thereafter pouring or rolling the blend thereof to uniform thickness on to a sheet or web of the non-adhesive water-tight material, or preferably foam material, of the dressing.

As an example of how to incorporate an active substance as for instance a medicament or an antiseptic into Na-CMC may be mentioned the dissolution of sodium carboxymethyl cellulose in distilled water to a concentration of 2% b.w. The active substance is then added, typically in an amount of 0.5-2% of the amount of Na-CMC, depending on the nature of the active substance.

The solution may then, optionally after a partial drying, be cast into sheets that are dried, or be spray-dried. Cast, dried sheets which are brittle may be easily ground to a desired particle size and particle size distribution whereas a spray-dried product will have the nature of dense fibres which are also easily ground.

If the active substance is insoluble in the sodium carboxymethyl cellulose solution, it is important to disperse it as evenly distributed minute particles or drops of solid or liquid (small compared to the desired particle size of the discontinuous phase) before the mixture is dried, so as to ensure an even distribution of the active substance in all of the Na-CMC-particles present after drying and comminution.

Another possibility resides in producing a homogenous mixture of finely divided sodium carboxymethyl cellulose and, calculated on the amount of Na-CMC, for instance 0.5-2% b.w. of the active substance. To this mixture water is added in an amount of expediently three time the weight of Na-CMC through spray nozzles under tumbling or vigorous shaking. The resulting gel is allowed to dry at a slightly elevated temperature or in vacuo. After grinding the product is comparable with the one described above.

In the following the dressing according to the invention will be described with reference to some examples of the production thereof. In the examples all parts and percentages are parts by weight and percentages by weight.

Example 1

To 100 parts by weight of pressure-sensitive adhesive material consisting of 36% styrene-isoprene-styrene block copolymer, 41% pinene tackifier resin and 22% liquid paraffin there is added 60 parts of sodium carboxymethyl cellulose powder into which 0.5% silver chloride has been incorporated as an antiseptic.

The material is then rolled out on sheets of a water-tight and water-proof plastic web and on the free surface thereof a protecting cover of siliconized paper is placed.

Example 2

To 100 parts of pressure-sensitive adhesive material consisting of polyisobutylene ("Vistanex"® LM) there is added 45 parts of guar gum powder and 60 parts of sodium carboxymethyl cellulose powder into which 1.2% of chlorohexidine are incorporated as an antiseptic.

Then the procedure of Example 1 is applied.

Example 3

To 100 parts of pressure-sensitive adhesive material consisting of 20 parts of styrene-isoprene-styrene block copolymer, 2 parts of dioctyl adipate, 30 parts of low molecular weight polyisobutylene and 48 parts of high molecular weight polyisobutylene there is added 80 parts of hydroxyethyl cellulose powder into which 1.0% povidone iodine has been incorporated into the powder particles.

Then the procedure of Example 1 is applied.

Example 4

To 100 parts of pressure-sensitive adhesive material consisting of 35% styrene-isoprene-styrene block copolymer, 45% of cyclopentadiene tackifier resin, 5% dioctyl adipate, 15% liquid paraffin there is added 70 parts of PVP (polyvinylpyrrolidone) into which 0.001% by weight of transforming growth factor $\beta$ (TGF $\beta$) has been added.

Then the procedure of Example 1 is applied.

**Claims**

1. A dressing which has a physiologically active substance incorporated therein and which comprises:

    (a) a sealing material comprising

    (i) a continuous phase comprising a tackifier, and

    (ii) a discontinuous phase dispersed in the continuous phase and comprising one or more water-soluble or water-swellable hydrocolloids, starches or cellulose derivatives or hydrophilic polymers, and

    (b) a non-adhesive water-tight film or other water-tight cover material on the surface of the dressing which in use is to be remote from the the skin of the patient to which the dressing is applied, the physiologically active substance being incorporated in the discontinuous phase.

2. A dressing as claimed in claim 1 in a form adapted for use in the attachment of incontinence or ostomy equipment, drainers and/or bags for collecting wound secretions.

3. A dressing as claimed in either of claims 1 and 2 wherein the continuous phase of the sealing material also comprises an elastomer, a plasticizer for the elastomer and/or other additives.

4. A dressing as claimed in any of the preceding claims wherein the physiologically active substance comprises an antiseptic agent.

5. A dressing as claimed in any one of claims 1 to 3 wherein the physiologically active substance comprises an antiinflammatory agent.

6. A dressing as claimed in any one of claims 1 to 3 wherein the discontinuous phase has an antiseptic agent and an antiinflammatory agent incorporated therein.

7. A dressing as claimed in any of the preceding claims wherein the sealing material is provided with a protective cover on the surface thereof remote from the non-adhesive water-tight film.

8. A method of producing a dressing as claimed in any of the preceding claims which comprises dissolving or dispersing a physiologically active substance evenly in a solution of the substance or substances forming the remainder of the discontinuous phase of the sealing material, subsequently drying the solution and, if necessary, comminuting the dried material to a desired particle size and sieving it to a desired particle size distribution, then kneading or otherwise incorporating the dry particles into a molten or heat-softened mass of components forming the continuous phase of the sealing material and thereafter pouring or rolling the blend thereof to uniform thickness on to a sheet or web of the non-adhesive water-tight material of the dressing.

9. A method as claimed in claim 8 wherein the solution of the substance or substances forming the remainder of the discontinuous phase is an aqueous solution.

**Revendications**

1. Pansement qui incorpore une substance physiologiquement active et qui comprend:

    (a) un matériau d'étanchéité comprenant

    (i) une phase continue comprenant un adhésif, et

    (ii) une phase discontinue dispersée dans la phase continue et comprenant un ou plusieurs hydrocolloïdes solubles ou gonflables dans l'eau, des dérivés d'amidon ou de cellulose ou des polymères hydrophiles, et

    (b) un film imperméable à l'eau et non adhésif, ou un autre matériau de couverture imperméable à l'eau, sur la face du pansement qui à l'emploi n'est pas tournée vers la peau du patient sur lequel le pansement doit être appliqué, la substance physiologiquement active étant incorporée dans la phase discontinue.

2. Pansement selon la revendication 1 dans une forme adaptée pour la fixation d'équipements d'incontinence ou d'ostomie, de drains et/ou de sacs collecteurs de suppurations de plaies.

3. Pansement selon la revendication 1 ou 2 dans lequel la phase continue du matériau d'étanchéité comprend en outre un élastomère, un plastifiant pour l'élastomère et/ou d'autres additifs.

4. Pansement selon une quelconque des revendications précédentes dans lequel la substance physiologiquement active comprend un agent antiseptique.

5. Pansement selon une quelconque des revendications 1 à 3 dans lequel la substance physiologiquement active comprend un agent antiinflammatoire.

6. Pansement selon une quelconque des revendications 1 à 3 dans lequel la phase discontinue incorpore un agent antiseptique et un agent antiinflammatoire.

7. Pansement selon une quelconque des revendications précédentes dans lequel le matériau d'étanchéité est pourvu, sur la face éloignée du film imperméable à l'eau et non adhésif, d'une couverture protectrice.

8. Procédé de fabrication d'un pansement selon une quelconque des revendications précédentes, consistant à dissoudre ou disperser une substance physiologiquement active de façon égale dans une solution de la substance ou des substances constituant le reste de la phase discontinue du matériau d'étanchéité, ensuite à sécher la solution et, au besoin, à pulvériser le matériau séché à une taille de particules désirée et le trier en une distribution de taille de particules désirée, ensuite à pétrir ou autrement incorporer les particules sèches dans une masse fondue, ou amollie par chaleur, de composants constituant la phase continue du matériau adhésif et ensuite à verser ou rouler ce mélange sous une épaisseur uniforme sur une feuille ou bande du matériau imperméable à l'eau et non adhésif du pansement.

9. Procédé selon la revendication 8 dans lequel la solution de la substance ou des substances constituant le reste de la phase discontinue est une solution aqueuse.

**Patentansprüche**

1. Ein Verband mit einem physiologisch aktiven Stoff und umfassend:
   (a) ein Abdichtungsmaterial bestehend aus
     (i) einer kontinuierlichen Phase mit einem Klebemittel, und
     (ii) einer diskontinuierlichen Phase, die in der kontinuierlichen Phase aufgelöst ist, und die aus einem oder mehreren wasserlöslichen oder wasserschwellbaren Hydrokolloiden, Stärke- oder Zellulosederivaten oder hydrophilen Polymeren, besteht, und
   (b) eine nicht-klebende, wasserdichte Folie oder ein anderes wasserdichtes Deckmaterial angeordnet auf der Oberfläche des Verbands, welche, wenn benutzt, der Haut des Patienten, der den Verband verwendet, abgewandt sein soll, indem der physiologisch aktive Stoff während der diskontinuierlichen Phase beigemischt wird.

2. Verband nach Anspruch 1 von einer Form, die zur Verwendung beim Festmachen von Inkontinenz- oder Ostomie-Ausrüstung, Abwässerungsausrüstung und/oder Beutel zur Aufsammlung von Wundesekretionen angepasst ist.

3. Verband nach jedem der Ansprüche 1 und 2, dadurch gekennzeichnet, dass die kontinuierliche Phase des Abdichtungsmaterials auch einen Elastomer, einen Weichmacher und/oder andere Additive umfasst.

4. Verband nach jedem der vorhergehenden Ansprüche, dadurch gekennzeichnet, dass das physiologisch aktive Material ein antiseptisches Mittel umfasst.

5. Verband nach jedem der Ansprüche 1 - 3, dadurch gekennzeichnet, dass das physiologisch aktive Material ein antiinflammatorisches Mittel umfasst.

6. Verband nach jedem der Ansprüche 1 - 3, dadurch gekennzeichnet, dass die diskontinuierliche Phase ein antiseptisches Mittel und ein antiinflammatorisches Mittel umfasst.

7. Verband nach jedem der vorhergehenden Ansprüche, dadurch gekennzeichnet, dass das Abdichtungsmaterial mit einer schützenden Decke auf der Oberfläche versehen ist, die der nicht-klebenden wasserdichten Folie abgewandt ist.

8. Verfahren zur Herstellung eines Verbands nach jedem der vorhergehenden Ansprüche, welches Verfahren die Auflösung oder Schlemmen eines physiologisch aktiven Materials gleichmässig in einer Lösung des Materials/der Materialien, das/die den restlichen Teil der diskontinuierlichen Phase des Abdichtungsmaterials ausmacht/ausmachen, umfasst, nachfolgendes Trocken der Lösung und, falls notwendig, eine Zerkleinerung des getrockneten Materials zu einer gewünschten Partikelgrösse, wonach es gesiebt wird, um die gewünschte Partikelgrösseverteilung zu erreichen, danach Einarbeitung der getrockneten Partikel durch Kneten oder auf eine andere Weise zur Bildung einer geschmolzenen oder durch Wärme weichgemachten Masse von Komponenten, die die kontinuierliche Phase des Abdichtungsmaterials ausmacht, und danach Auslegung der Mischung durch Giessen oder Walzen in gleichförmiger Dicke auf ein Tuch oder ein Gewebe vom nichtklebenden, wasserdichten Material des Verbands.

9. Verfahren nach Anspruch 8, dadurch gekennzeichnet, dass das Material oder die Materialen, das/die den restlichen Teil der diskontinuierlichen Phase ausmacht/ausmachen eine wässerige Lösung ist.